(19) Europäisches Patentamt
European Patent Office
Office européen des brevets

(11) **EP 3 012 671 A1**

(12) **EUROPEAN PATENT APPLICATION**

(43) Date of publication:
**27.04.2016 Bulletin 2016/17**

(51) Int Cl.:
***G01V 9/00*** *(2006.01)*    ***E21B 49/00*** *(2006.01)*
***G01N 33/28*** *(2006.01)*    *G01V 1/40* *(2006.01)*

(21) Application number: **14290323.6**

(22) Date of filing: **22.10.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Geoservices Equipements**
**95971 Roissy en France (FR)**

(72) Inventors:
• **Ringer, Maurice**
**60260 Lamorlaye (FR)**

• **Lessi, Jacques**
**78300 Poissy (FR)**

(74) Representative: **Leonori, Céline**
**Etudes et Productions Schlumberger**
**Intellectual Property Department**
**1, rue Henri Becquerel**
**B.P. 202**
**92142 Clamart Cedex (FR)**

(54) **System and method for estimating properties of geological formations drilled using underreamer**

(57)    Devices and methods for estimating properties of geological formations drilled with a drill bit (16) and an underreamer (18) are provided. A method according to the disclosure includes receiving a set of mixed well-logging data corresponding to a geological formation at multiple depths of a wellbore drilled using a bottom hole assembly (28) that includes a drill bit and a first underreamer. The drill bit and the first underreamer are spaced a first distance (76) apart from one another. The method includes separating the set of mixed well-logging data into first well-logging data corresponding to the geological formation at depths drilled by the drill bit (16) and second well-logging data corresponding to the geological formation at depths reamed by the first underreamer (18).

FIG. 3

**Description**

**BACKGROUND**

This disclosure relates to system and method for estimating properties of geological formations of a wellbore drilled with a drill bit and an underreamer.

This section is intended to introduce the reader to various aspects of art that may be related to various aspects of the present techniques, which are described and/or claimed below. This discussion is believed to be helpful in providing the reader with background information to facilitate a better understanding of the various aspects of the present disclosure. Accordingly, it should be understood that these statements are to be read in this light, and not as an admission of any kind.

Identifying the properties of the geological formation may provide information about the likely presence or absence of hydrocarbons and/or the state of a drilling operation. Some useful formation properties may include a specific energy of the formation, a volume of the formation fluid released from the geological formation, the quantities of particular chemicals (e.g., alkanes such as methane or ethane, or hydrogen sulfide) within the formation fluid, and/or a composition of the formation. The specific energy of the formation relates to the energy being used to drill the well through various depths. Depths where more energy is used to drill through the geological formation are understood to have a comparatively higher specific energy, while depths where less energy is used to drill through the geological formation are understood to have a comparatively lower specific energy. The composition of the formation may include information relating to materials in the various depths of the well. As the well is drilled, cuttings and formation fluids are removed from the well. At the surface, these materials may be examined and analyzed to determine properties about the geological formation at various depths.

Correlating the formation properties mentioned above with the depths that they occur may be relatively straightforward when the well is drilled with a drill bit. However, when drilling with a bottom hole assembly (BHA) that includes both a drill bit and one or several underreamer to further open the wellbore, obtaining properties of the geological formation using the same techniques may not produce accurate results. The underreamer is generally disposed above the drill bit with a certain distance and has a greater cross-section area of cutting in order to enlarge the borehole. As such, the geological formation is cut simultaneously by the drill bit and the underreamer during drill at different respective depths. Formation fluid may also be released from the geological formation simultaneously at the drill bit depth and at the underreamer depth. Thus, the cuttings and/or the formation fluid obtained at surface at a given time may be a combination of cuttings and/or formation fluid from two different depths. Accordingly, the properties of the geological formation measured at a given time may be a combination of components coming from the geological formation drilled by the drill bit and by the underreamer. As such, the measured properties of the geological formation may not reflect the actual properties of the geological formation at a particular depth.

**SUMMARY**

A summary of certain embodiments disclosed herein is set forth below. It should be understood that these aspects are presented merely to provide the reader with a brief summary of these certain embodiments and that these aspects are not intended to limit the scope of this disclosure. Indeed, this disclosure may encompass a variety of aspects that may not be set forth below.

Embodiments of the disclosure relate to estimating properties of geological formations drilled with a drill bit and an underreamer. A method includes receiving a set of mixed well-logging data corresponding to a geological formation at multiple depths of a wellbore drilled using a bottom hole assembly (BHA) that includes a drill bit and a first underreamer. The drill bit and the first underreamer are spaced a first distance apart from one another. The method also includes separating the set of mixed well-logging data into first well-logging data corresponding to the geological formation at depths drilled by the drill bit and second well-logging data corresponding to the geological formation at depths reamed by the first underreamer.

In another example, a downhole device includes a bottom hole assembly (BHA) that includes a drill bit and a first underreamer. The drill bit and the first underreamer are spaced a first distance apart from one another. The downhole device also includes a controller. The controller includes a processor programmed to receive a set of mixed well-logging data corresponding to a geological formation at multiple depths of a wellbore drilled using the BHA and to decompose the set of mixed well-logging data into first well-logging data corresponding to the geological formation at depths drilled by the drill bit and second well-logging data corresponding to the geological formation at depths reamed by the first underreamer.

Moreover, a tangible, non-transitory, computer-readable medium includes instructions to receive a set of mixed well-logging data corresponding to a property of a geological formation at multiple depths of a wellbore drilled using a bottom hole assembly

(BHA) that includes a drill bit and a first underreamer. The drill bit and the first underreamer are spaced a first distance apart from one another. The tangible, non-transitory, computer-readable medium also includes instructions to deconvolve the set of mixed well-logging data into first well-logging data corresponding to a first component of the property of the geological formation at the multiple depths attributable to the drill bit and a second component of the property of the geological formation at the multiple depths attributable to the first underreamer. The tangible, non-transitory, computer-readable medium further includes instructions to generate an output of a first component and a second component.

Various refinements of the features noted above may be undertaken in relation to various aspects of the present disclosure. Further features may also be incorporated in these various aspects as well. These refinements and additional features may exist individually or in any combination. For instance, various features discussed below in relation to one or more of the illustrated embodiments may be incorporated into any of the above-described aspects of the present disclosure alone or in any combination. The brief summary presented above is intended to familiarize the reader with certain aspects and contexts of embodiments of the present disclosure without limitation to the claimed subject matter.

## BRIEF DESCRIPTION OF THE DRAWINGS

Various aspects of this disclosure may be better understood upon reading the following detailed description and upon reference to the drawings in which:

FIG. 1 is a schematic diagram of a drilling system that includes a control system for estimating properties of a geological formation drilled with a bottom hole assembly (BHA) that includes a drill bit and an underreamer, in accordance with an embodiment;

FIG. 2 is a schematic diagram of another drilling system that includes a control system for estimating properties of a geological formation drilled with a BHA that includes a drill bit and an underreamer, in accordance with an embodiment;

FIG. 3 is a schematic view of the BHA of FIG. 1 for drilling a geological formation and a corresponding parameter as a function of depth of the geological formation, in accordance with an embodiment;

FIG. 4 is a flow chart of a method for estimating specific energy of a geological formation at multiple depths drilled with the BHA of FIG. 1, in accordance with an embodiment;

FIG. 5 is a chart showing specific energy as a function of depth, in accordance with an embodiment;

FIG. 6 is a flow chart of a method for estimating quantity of the formation fluid from the geological formation at multiple depths drilled with the BHA of FIG. 1, in accordance with an embodiment;

FIG. 7 is a chart showing depths of the bit and the underreamer of the BHA of FIG. 1 as a function of time, in accordance with an embodiment;

FIG. 8 is a chart showing depths of the bit and the underreamer of the BHA of FIG. 1 as a function of time, in accordance with an embodiment;

FIG. 9 is a chart showing measurement of a property formation fluid as a function of time, in accordance with an embodiment;

FIG. 10 is a chart showing a property of formation fluid as a function of depth, in accordance with an embodiment; and

FIG. 11 is a flow chart of a method for determining a property of a geological formation at multiple depths drilled with the BHA of FIG. 1, in accordance with an embodiment.

## DETAILED DESCRIPTION

One or more specific embodiments of the present disclosure will be described below. These described embodiments are examples of the presently disclosed techniques. Additionally, in an effort to provide a concise description of these embodiments, features of an actual implementation may not be described in the specification. It should be appreciated that in the development of any such actual implementation, as in any engineering or design project, numerous implementation-specific decisions may be made to achieve the developers' specific goals, such as compliance with system-related and business-related constraints, which may vary from one implementation to another. Moreover, it should be appreciated that such a development effort might be complex and time consuming, but would be a routine undertaking of design, fabrication, and manufacture for those of ordinary skill having the benefit of this disclosure.

When introducing elements of various embodiments of the present disclosure, the articles "a," "an," and "the" are intended to mean that there are one or more of the elements. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features.

Properties of a geological formation of a wellbore may be obtained through well logging. As noted above, well-logging data may be obtained via measurement or monitoring of a downhole tool within the wellbore and/or via analysis of cuttings of the geological formation and formation fluid carried by drilling fluid at surface. In the case of drilling by a BHA that includes only a drill bit, the well-logging data obtained at a given time may correlate with the geological formation at a particular depth of the wellbore and, accordingly, may be used to derive properties of the geological formation at that particular depth in a relatively straightforward manner.

However, in the case of drilling by a BHA that includes both a drill bit and an underreamer, the well-logging data obtained at a given time may have more complicated correlation with the geological formation at a particular depth of the wellbore. Indeed, the underreamer may be spaced apart from the drill bit with a distance and may have a greater cross-sectional area of cutting than the drill bit. As such, the geological formation may be cut simultaneously by the drill bit, at one depth, and the underreamer during drill, at another depth, and the formation fluid may also be released from the geological formation simultaneously at the drill bit and at the underreamer at the two different depths. Accordingly, the well-logging data obtained at a given time may include components from both the drill bit and the underreamer that cut the geological formation and/or release the formation fluid stored in the geological formation at different depths. In other words, in the case of drilling by the BHA that includes both the drill bit and the underreamer, the well-logging data is "mixed" with components attributable to both the drill bit and the underreamer.

To obtain or estimate actual properties of the geological formation at a particular depth, techniques are provided herein for separating the properties of the geological formation derived from the mixed well-logging data into a component (or components) coming from the geological formation drilled by the drill bit and another component (or other components) coming from the geological formation drilled by the underreamer. More specifically, embodiments of the present disclosure may include receiving a set of mixed well-logging data corresponding to a geological formation at multiple depths of a wellbore drilled using a BHA that includes a drill bit and an underreamer, and separating the set of mixed well-logging data into first well-logging data corresponding to the geological formation at depths drilled by the drill bit and second well-logging data corresponding to the geological formation at depths cut by the underreamer. The set of mixed well-logging data may include measurement made by a downhole tool below surface, such as parameters related to specific energy of the geological formation at the multiple depths, and/or measurement made by a measurement device at surface, such as parameters related to formation cuttings, formation fluid released from the wellbore, or drilling fluid exiting the wellbore. In certain embodiments, separating the set of mixed well-logging data may include solving a set of equations for a property of the geological formation at the multiple depths of the wellbore by solving a matrix equation.

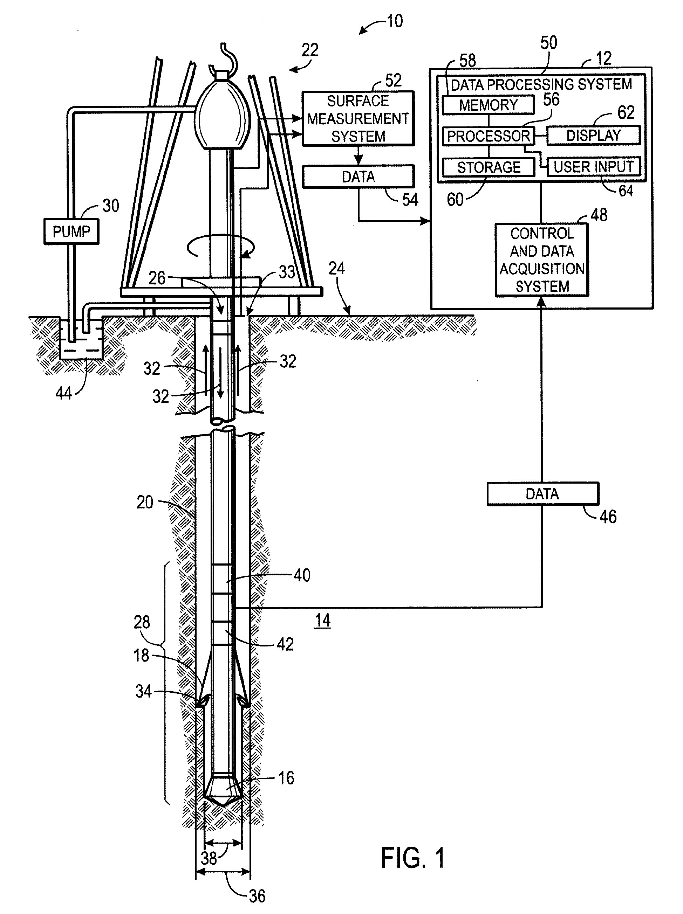

With the foregoing in mind, FIG. 1 illustrates a drilling system 10 that includes a control system 12 for estimating properties of a geological formation 14 drilled with a drill bit 16 and an underreamer 18. The drilling system 10 may be used to drill a well (e.g., a wellbore 20) into the geological formation 14 and obtain logging measurements useful to identify characteristics of the well. In the drilling system 10, a drilling rig 22 at surface 24 may rotate a drill string 26 having a bottom-hole assembly (BHA) 28 at its lower end.

As illustrated in FIG. 1, the BHA 28 includes the drill bit 16 and the underreamer 18. The drill bit 16 is located on the downhole end of the BHA and configured to drill or cut the geological formation about the bottom of the wellbore 20. The underreamer 18 is disposed above (e.g., away from the downhole end of the BHA) the drill bit 16 and includes multiple elongated arms 34, which may be activated (e.g., by hydraulics, electric motor, etc.) to ream or cut the geological formation at the downhole end of the underreamer 18. The underreamer 18 may have a greater cross-sectional area of cutting than the drill bit 16 (e.g., the diameter 36 of the cross-sectional area of cutting for the underreamer 18 is greater than the diameter 38 of the cross-sectional area of cutting for the drill bit 16). In some embodiments, the BHA 28 may include more than one underreamer 18, which may be disposed above one another and may have different cross-sectional areas of cutting.

As the BHA 28 is rotated, a drilling fluid pump 30 is used to pump drilling fluid 32, which may be referred to as "mud" or "drilling mud," downward through the center of the drill string 26 in the direction of the arrow to the drill bit 16. The drilling fluid 32, which is used to cool and lubricate the drill bit 16, exits the drill string 26 through the drill bit 16. The drilling fluid 32 then carries drill cuttings away from the bottom of the wellbore 20 as it flows back to the surface 24, as shown by the arrows through an annulus 33 between the drill string 26 and the formation 14. In addition, as the drilling fluid 23 flows through the annulus 33 between the drill string 26 and the formation 14, the drilling fluid 32 may begin to invade and mix with fluid stored in the formation, which may be referred to as formation fluid (e.g., natural gas, or oil, or a combination thereof). At the surface 24, return drilling fluid 32 is filtered and conveyed back to a mud pit 44 for reuse.

The BHA 28 may also include one or more downhole tools. The downhole tools may collect a variety of information relating to the geological formation 14 and/or the state of drilling of the well. For instance, a measurement-while-drilling (MWD) tool 40 may measure certain drilling parameters, such as the temperature, pressure, orientation of the drilling units (e.g., the drill bit 16 and the underreamer 18), angular speed of the drilling units, weight applied to the drilling units, torque generated by the drilling units, distance drilled per unit angular rotation (the depth-of-cut), rate of penetration, and so forth. Likewise, a logging-while-drilling (LWD) tool 42 may measure the physical properties of the geological formation 14, such as density, porosity, resistivity, lithology, and so forth. The MWD tool 40 and the LWD tool 42, as illustrated, are disposed above the underreamer 18.

The MWD tool 40 and/or the LWD tool 42 may collect a variety of data 46 that may be stored and processed in the BHA 28 or, as illustrated in FIG. 1, may be sent to the surface 24 for processing. The data 46 may be sent via a control and data acquisition system 48 to a data processing system 50 of the control system 12. The control and data acquisition system 48 may receive the data 46 in any suitable way. In one example, the control and data acquisition system 48 may transfer the data 46 via electrical signals pulsed through the geological formation 14 or via mud pulse telemetry using the drilling fluid 32. In another example, the data 46 may be retrieved directly from the MWD tool 40 and/or the LWD tool 42 upon return to the surface 24.

The drilling fluid 32, or the drilling cuttings and/or formation fluid carried by the drilling fluid 32, may be analyzed upon return to the surface 24, for example, by a surface measurement system 52. The surface measurement system 52 may include any suitable device to measure the physical and/or chemical properties of the geological formation 14, such as density, porosity, resistivity, lithology, quantity of the formation fluid stored thereof, presence and quantities of certain chemicals therewithin, and so forth. The surface measurement system 52 may also be directly coupled to an above-the-surface portion of the drilling rig 22 to measure certain drilling parameters, such as the temperature, pressure, orientation of the drilling units (e.g., the drill bit 16 and the underreamer 18), weight applied to the drilling units, torque generated by the drilling units, distance drilled per unit angular rotation (the depth-of-cut), and so forth. Data 54 collected by the surface measurement system 52 may be processed in the surface measurement system 52 and sent via the control and data acquisition system 48 to the data processing system 50, or may be sent via the control and data acquisition system 48 to the data processing system 50 directly. Likewise, the control and data acquisition system 48 may receive the data 54 in any suitable way. The data 46, 52, or a portion thereof, may be generally referred to as well-logging data of the drilling system 10. As noted above, the data 46, 52 may be mixed well-logging data corresponding to the geological formation 14 at multiple depths of the wellbore 20 drilled with the drill bit 16 and the underreamer 18.

The data processing system 50 may include a processor 56, memory 58, storage 60, a display 62, and/or a user input 64. The data processing system 50 may use the data 46, 54 to determine various properties of the well, including the geological formation 14. More specifically, as will be discussed in greater detail below, the data processing system 50 may separate the properties of the geological formation 14 derived from the mixed well-logging data (e.g., the data 46, 54) into a component (or components) coming from the geological formation 14 drilled by the drill bit 16 and another component (or other components) coming from the geological formation drilled by the underreamer 18. To process the data 46, 54, the processor 56 may execute instructions stored in the memory 58 and/or storage 60. As such, the memory 58 and/or the storage 60 of the data processing system 50 may be any suitable article of manufacture that can store the instructions. The memory 46 and/or the storage 60 may be ROM memory, random-access memory (RAM), flash memory, an optical storage medium, or a hard disk drive, to name a few examples. The display 62 may be any suitable electronic display that can display the logs and/or other information relating to properties of the well such as the separated components coming from the geological formation 14 drilled by the drill bit 16 and the underreamer 18. The user input 64 may be any suitable device that can be used by a user to input instructions, parameters, boundary conditions, or the like for deriving, separating, or decomposing the properties of the geological formation 14. The user input 64 may include a mouse, a keyboard, a touchpad, a touch screen, a voice recognition system, or the like. It should be appreciated that, although the data processing system 50 is shown by way of example as being located at the surface 54, the data processing system 50 may be located under the surface 54 (e.g., in the MWD tool 40 and/or the LWD tool 42).

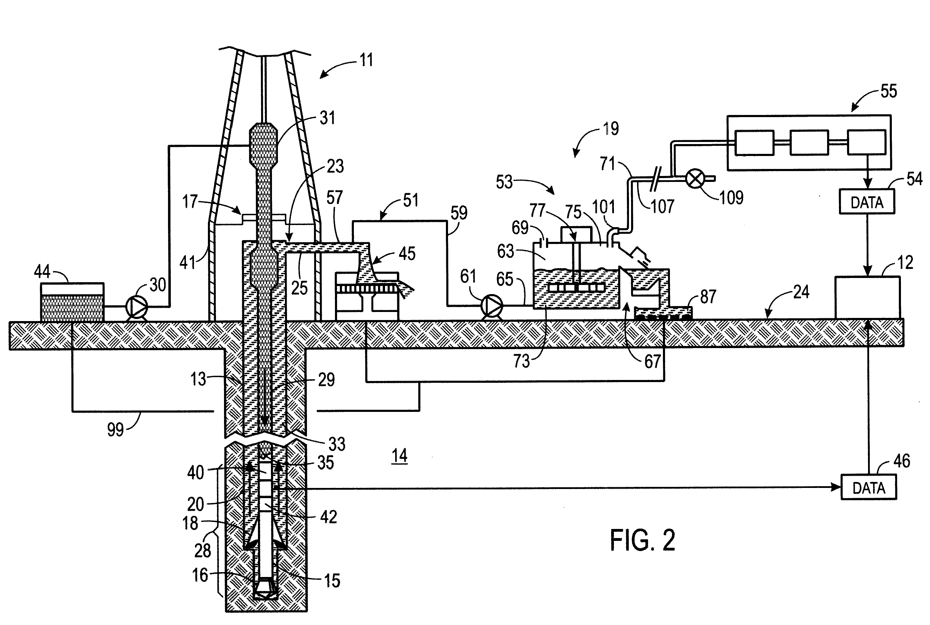

FIG. 2 represents another example of a drilling system 11 that includes the control system 12 for estimating properties of the geological formation 14 drilled with the drill bit 16 and the underreamer 18. The drilling system 11 includes a drilling pipe 13 in the wellbore 20 through which a rotating drilling tool 15 extends. The drilling pipe 13 includes, in the region of the surface 24, a well head 23 which is provided with a pipe 25 for discharging the drilling fluid 32. The drilling tool 15 includes the BHA 28, a drilling assembly 29 and a liquid injection head 31. The BHA 28 includes the drill bit 16 and the underreamer 18 and may also include the MWD tool 40 and/or the LWD tool 42. The BHA 28 is assembled in the lower portion of the drilling assembly 29 and is positioned at the bottom of the drilling pipe 13.

The drilling assembly 29 includes an assembly of hollow drilling tubes also called drill string. These tubes delimit an internal space 35 which allows the drilling fluid 32 to be conveyed from the surface 24 to the drill bit 16. The liquid injection head 31 is coupled to (e.g., screwed onto) the upper portion of the drilling assembly 29.

The drilling system 11 also includes a surface installation 17. The surface installation 17 includes means 41 for supporting and driving the drilling tool 15 in rotation, the mud pit 44, and the drilling fluid pump 30 to pump the drilling fluid 32 from the mud pit 44 to the liquid injection head 31. The drilling fluid 32 is then directed to the discharge pipe 25.

The surface installation 17 also includes a vibrating sieve 45. The vibrating sieve 45, also called shaker, collects the drilling fluid 32 charged with drilling residues (e.g., the drilling cuttings and/or formation fluid carried by the drilling fluid 32) which is discharged from the discharge pipe 25 and separates the fluid from the solid drilling residues. The drilling fluid 32 once separated from the solid drilling residues, is taken away from the well. The drilling fluid 32 will afterwards be stored and/or recycled and/or treated. The drilling cuttings may also be cleaned and analyzed.

The drilling system 11 also includes an analysis assembly 19. The analysis assembly 19 includes a sampling device 51 coupled to the discharge pipe 25 for sampling the drilling fluid 32, a gas extraction device 53, and an analysis device 55 for analyzing and quantifying the extracted gases. The sampling device 51 includes a liquid sampling head 57 which is tapped into the discharge pipe 25, a connection tube 59, and a peristaltic pump 61 whose flow rate can be adjusted. The extraction device 53 includes a vessel 63, a mud supply pipe 65 for conveying the drilling fluid 32 into the vessel 63, a pipe 67 for discharging the drilling fluid 32 from the vessel 63, an inlet 69 for introducing a carrier gas into the vessel 63, and a pipe 71 for extracting the extracted gases from the vessel 63.

The vessel 63 is formed by a sealed receptacle whose inner volume is, for example, between 0.4 and 3 liters. This vessel 63 includes a lower portion 73 in which the drilling fluid 32 circulates and an upper portion 75 which has a gaseous cap. The vessel 63 is further provided with an agitator 77 which is immersed in the drilling fluid 32.

The mud supply pipe 65 extends between the outlet of the peristaltic pump 61 and an inlet opening which is arranged in the lower portion 73 of the vessel 63. The mud supply pipe 65 may be provided with a heater for heating the drilling fluid 32 in order to bring the temperature of this mud to values of between 25 and 120 °C., for instance between 60 and 90 °C.

The discharge pipe 67 extends between an overflow passage which is arranged in the upper portion 75 of the vessel 63 and a retaining vessel 87 which is intended to receive the drilling fluid 32 which is discharged from the extraction device 53. It includes a siphon in order to prevent gas from being introduced into the upper portion 75 of the vessel 63 via the discharge pipe 67. Gas is therefore introduced into the vessel 63 via the carrier gas introduction inlet 69. Carrier gas is an inert gas that does not include any of the constituent that should be detected by the quantification device, such as air or Helium for example. The drilling fluid 32 which is collected in the retaining vessel 87 is recycled towards the mud pit 44 via a mud recirculation pipe 99.

The gas extraction pipe 71 extends between an extraction opening 101, which is arranged in the upper portion 75 of the vessel 63, and the analysis device 55. The gas extraction pipe 71 includes a transport line 107 which is provided with volume flow control means and suction means 109. The transport line 107 connects the vessel 63 which is arranged in the vicinity of the well head 23, in the explosive zone, to the analysis device 55 which is arranged with spacing from the well head 23 in a non-explosive zone, for example, in a pressurized cabin. However, the gas analysis device 55 may be arranged also in the vicinity of the well head. The transport line 107 is normally at least 10 meters long. The transport line 107 may be made of polymer material such as PTFE or THV, as explained in patent No. US7232548 hereby incorporated by reference. The suction means 109 includes a vacuum pump which allows the gases extracted from the vessel 63 to be conveyed, by means of suction, to the analysis device 55.

Similar to the drilling system 10 of FIG. 1, in the drilling system 11 of FIG. 2, the MWD tool 40 and/or the LWD tool 42 may collect a variety of data 46 that may be stored and processed in the BHA 28 or may be sent to the control system 12 (e.g., the data processing system 50) for processing. Also, the data 54 collected by the analysis device 55 may be processed in the analysis device 55 and then sent to the control system 12 (e.g., the data processing system 50), or may be sent to the control system 12 (e.g., the data processing system 50) directly. Again, the data 46, 52 may be mixed well-logging data corresponding to the geological formation 14 at multiple depths of the wellbore 20 drilled with the drill bit 16 and the underreamer 18.

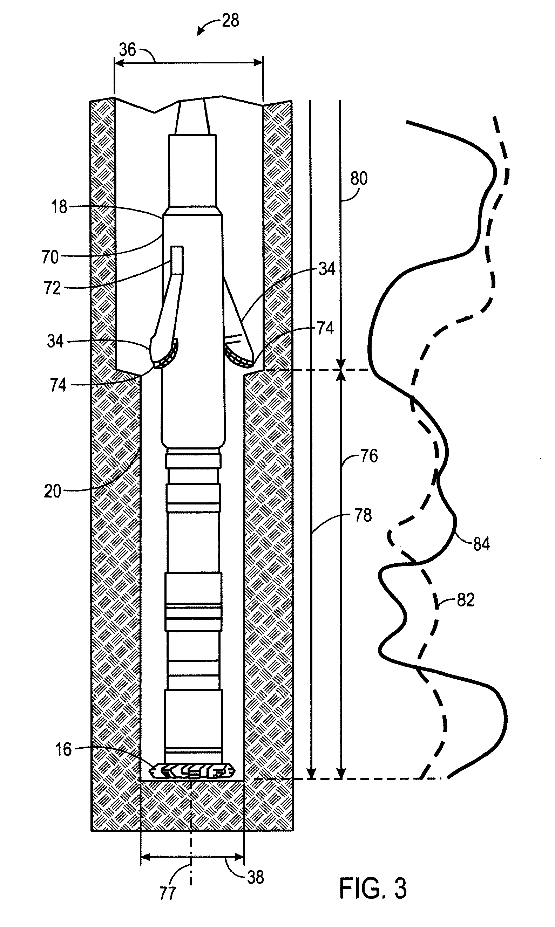

It should be noted that the techniques described herein may be applied to any mixed well-logging data that includes data relating to a BHA 28 that includes a drill bit 16 and an underreamer 18. It should also be noted that the techniques described herein may be applied to a BHA 28 that includes a drill bit 16 and multiple underreamers 18. FIG. 3 illustrates an embodiment of the BHA 28 that includes the drill bit 16 and the underreamer 18, as well as data of an example property, specific energy, of the geological formation 14 at different depths of the wellbore 20. The specific energy is referred to as the amount of energy or work used to break unit volume of the geological formation 14 (e.g., rock). The specific energy may provide information on the compressive strength of the geological formation 14 at a given depth of the wellbore 20.

As illustrated, the underreamer 18 includes a body 70 and the multiple elongated arms 34. It should be noted that the underreamer 18 may include any suitable number of the arms 34. Each arm 34 is pivotally connected at a pivot point 72 to the body 70 and includes a cutting element 74 at the downhole end of the arm 34. The arms 34 may be recessed into the body 70 completely or partially and configured to open up with respect to the pivotal pints 72 to their cutting positions (e.g., by hydraulics or an electric motor) during drilling. As illustrated, the diameter 36 of the cross-sectional area of cutting for the underreamer 18 is greater than the diameter 38 of the cross-sectional area of cutting for the drill bit 16. In addition, the underreamer 18 is positioned above (e.g., away from the downhole end) the drill bit 16 with a distance 76 (along a vertical axis 77 of the wellbore 20) spaced apart therefrom. As such, when the drill bit 16 is cutting at a depth 78 relative to the surface 24 of the wellbore 20, the underreamer is cutting at another depth 80, which is less than the depth 78 by the distance 76 between the drill bit 16 and the underreamer 18. Therefore, the specific energy measured at a given time is a combination of the specific energy of the geological formation 14 at the depth 78, which is cut by the drill bit 16, and the specific energy of the geological formation 14 at the depth 80, which is cut by the underreamer 18.

FIG. 3 also illustrates a first curve 82 representing an example of a measured property as a function of depth. A second curve 84 represents an example of actual (or true) property of the geological formation 14 as a function of depth. As noted above, because the measured property includes contributions from both the drill bit 16 and the underreamer 18, which are at different depths, there is a discrepancy between the first curve 82 and the second curve 84.

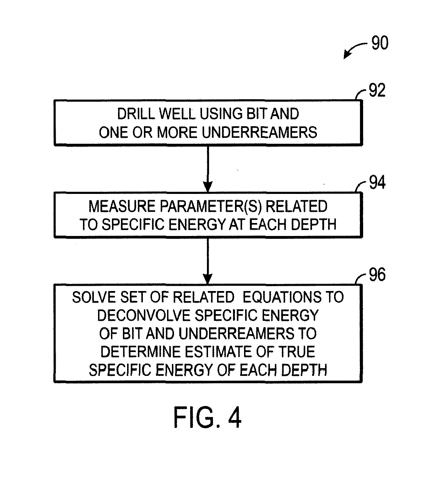

FIG. 4 illustrates a method 90 for estimating the specific energy of the geological formation 14 at multiple depths of the wellbore 20 drilled with the BHA 28 that includes the drill bit 16 and the underreamer 18. The method 90 may start with drilling the wellbore 20 using the drill bit 16 and one or more underreamers (block 92). At each depth of the wellbore 20, one or more parameters (e.g., the total weight, $W$, and total torque, $T$, of the BHA 28) related to the specific energy of the geological formation 14 may be measured (block 94). As noted above, the parameters may be measured below the surface 24 by one or more downhole.tools, such as the MWD tool 40 of the BHA 28, and/or at the surface 24 by the surface measurement system 52.

At each depth, the specific energy may be expressed in an equation, as discussed in more detail below, as a function of the measured parameters. Also as noted above, the specific energy at each depth generally includes components coming from the drill bit 16 at that depth and the underreamer 18 at a smaller depth because of a distance between the drill bit 16 and the underreamer 18 (e.g., the distance 76 as illustrated in FIG. 3). As such, a set of related equations about the specific energy may be constructed. For example, if the drill bit 16 drills from a first depth to a second depth when the second depth is greater than the first depth by the distance between the drill bit 16 and the underreamer 18, the specific energy at the second depth is related to the specific energy at the first depth because the underreamer 18 is at the first depth when the drill bit 16 advances to the second depth. As such, the equations for the specific energies at the first and the second depths are related. The method 90 thus includes solving the set of related equations to deconvolve specific energy components coming from the drill bit 16 and the underreamer 18 to determine an estimate of the actual (true) specific energy of the geological formation 14 at each depth (block 96).

More specifically, the specific energy of the geological formation 14 may be calculated or derived from the well-logging data. For example, the specific energy, $\varepsilon$, may be expressed as the following:

$$\varepsilon = \frac{W}{A} + \frac{T}{A\,D} \qquad (1),$$

where $W$ is the total axial weight (e.g., the weight along the vertical axis 77) on the cutting structure, $T$ is the downhole torque, $A$ is the cross-sectional area of the geological formation 14 (e.g., rock) being removed, and $D$ is the distance drilled per unit angular rotation (the depth-of-cut).

When drilling the wellbore 20 using the BHA that includes the drill bit 16 and the underreamer 18, the bit 16 and the underreamer 18 are cutting the geological formation 14 (e.g., rock) at different depths, for example, the depth 78 and the depth 80 as illustrated in FIG. 3. Accordingly, the specific energy at the drill bit 16 is:

$$\varepsilon(d) = \frac{W_B}{A_B} + \frac{T_B}{A_B D} \qquad (2),$$

where $W_B$ is the axial weight (e.g., the weight along the vertical axis 77) on the drill bit 16, $T_B$ is the torque generated by the drill bit 16, $A_B$ is the cross-sectional area of the geological formation 14 (e.g., rock) being cut by the drill bit 16, $D$ is the distance drilled per unit angular rotation (the depth-of-cut), $d$ is the depth of the drill bit 16 (e.g., the depth 78 as illustrated in FIG. 3), and $\varepsilon(d)$ is the specific energy of the geological formation 14 (e.g., rock) at the depth $d$.

Likewise, the specific energy at the underreamer 18 is:

$$\varepsilon(d - \delta) = \frac{W_U}{A_U} + \frac{T_U}{A_U D} \tag{3},$$

where $W_U$ is the axial weight (e.g., the weight along the vertical axis 77) on the underreamer 18, $T_U$ is the torque generated by the underreamer 18, $A_U$ is the cross-sectional area of the geological formation 14 (e.g., rock) being reamed or cut by the underreamer 18, $D$ is the distance drilled per unit angular rotation (the depth-of-cut), $\delta$ is the distance between the drill bit 16 and the underreamer 18 (e.g., distance 76 as illustrated in FIG. 3), $d-\delta$ is the depth of the underreamer 18 (e.g., the depth 80 as illustrated in FIG. 3), and $\varepsilon(d-\delta)$ is the specific energy of the geological formation 14 (e.g., rock) at the depth $d-\delta$ ($\delta$ being the distance between the bit 16 and the underreamer 18).

As noted above, the BHA 28 may include one or more downhole tools, such as the MWD tool 40, and/or the surface measurement system 52 may measure certain drilling parameters, such as the weight applied to the drilling units and the torque generated by the drilling units. Typically, the total weight, $W$, applied and the total torque, $T$, generated downhole are measured, which include components from both the drill bit 16 and the underreamer 18:

$$W = W_B + W_U \tag{4};$$

and

$$T = T_B + T_U \tag{5}.$$

Combining Equations (2), (3), (4), and (5) gives:

$$W + \frac{T}{D} = A_B \varepsilon(d) + A_U \varepsilon(d - \delta) \tag{6}.$$

Thus, the measurements of the total weight, $W$, and the total torque, $T$, includes two components coming from the specific energy of the geological formation 14 (e.g., rock) at the drill bit 16 and the underreamer 18, which are at different depths, $d$ and $d-\delta$, respectively.

Equation (6) may be written for different depths. For example, when the drill bit 16 starts to drill the geological formation 14, the underreamer 18 is not yet drilling. At this first depth (e.g., depth 1) of the drill bit 16, Equation (6) becomes:

$$W(1) + \frac{T(1)}{D(1)} = A_B \varepsilon(1) \tag{7}.$$

At any depth (e.g., depth 2) where the underreamer 18 is not yet drilling, Equation (6) may be written similarly to Equation (7). As the drill bit 16 drills downward further in the wellbore 20 to a depth (e.g., depth 3) that is substantially the same as the distance, $\delta$, between the drill bit 16 and the underreamer 18, the underreamer 18 starts to drill the geological formation 14. Thus, the underreamer 18 is now at depth 1, and Equation (6) becomes:

$$W(3) + \frac{T(3)}{D(3)} = A_B \varepsilon(3) + A_U \varepsilon(1) \tag{8}.$$

At any depth that is greater than depth 3 (e.g., depth 4, 5, 6, and so forth), both the drill bit 16 and the underreamer 18 are drilling the geological formation 14, and Equation (6) may be written similarly to Equation (8). Accordingly, a set of equations (e.g., including Equations (7) and (8)) based on Equation (6) may be constructed for different depths.

Because the total weight, *W,* the total torque, *T,* the distance drilled per unit angular rotation, *D,* and the cross-sectional area of the geological formation 14 (e.g., rock) cut by the drill bit 16, $A_B$, and the underreamer 18, $A_U$, respectively, may be measured by the downhole tools (e.g. the MWD tool 40) below the surface 24 and/or the surface measurement device 52 at the surface 24, the specific energy of the geological formation 14 (e.g., rock) may be obtained at any depth (e.g., depth 1, 2, 3, 4, 5, 6, and so forth). As such, the set of mixed well-logging data (e.g., the measured total weight, *W,* and the total torque, *T*) corresponding to the specific energy of the geological formation 14 at multiple depths of the wellbore 20 may be separated into the specific energy components coming from the drill bit 16 and the underreamer 18 without measuring or knowing how the total weight, *W,* and the total torque, *T* is split between the drill bit 16 and the underreamer 18.

In accordance with the present disclosure, solving for the specific energy at different depths may be done using any suitable methods. For example, a matrix equation may be constructed based on Equation (6), or more specifically, Equations (7), (8), and so forth:

$$\begin{bmatrix} W(1) + T(1)/D(1) \\ W(2) + T(2)/D(2) \\ W(3) + T(3)/D(3) \\ \vdots \\ W(d) + T(d)/D(d) \end{bmatrix} = \begin{bmatrix} A_B & 0 & & & & & \\ 0 & A_B & 0 & & & & \\ A_U & 0 & A_B & 0 & & & \\ 0 & \ddots & \ddots & \ddots & \ddots & & \\ & & 0 & A_U & 0 & A_B & 0 \\ & & & 0 & A_U & 0 & A_B \end{bmatrix} \begin{bmatrix} \varepsilon(1) \\ \varepsilon(2) \\ \varepsilon(3) \\ \vdots \\ \varepsilon(d) \end{bmatrix} \qquad (9).$$

Other methods such as least square methods for instance may be used for solving the linear system.

In Equation (9), the set of mixed well-logging data (e.g., the measured total weight, *W,* the total torque, *T,* and the distance drilled per unit angular rotation, *D*) at multiple depths (e.g., depth 1, 2, 3, ... *d*) may be measured and written as a first vector on the left side. The right side of Equation (9) includes a matrix and a second vector. The matrix includes non-zero elements of the cross-sectional area of the geological formation 14 (e.g., rock) cut by the drill bit 16, $A_B$, and the underreamer 18, $A_U$, respectively, which may be measured or known. As such, the second vector including the specific energy of the geological formation 14 at the multiple depths (e.g., depth 1, 2, 3,...,*d*) may be determined using linear algebra.

As shown in Equation (9), each of the first two rows includes only $A_B$ as the only non-zero element as at depths 1 and 2, only the drill bit 16 is cutting the geological formation 14. The third row of the matrix includes both $A_B$ and $A_U$ as the non-zero elements as at depth 3, both the drill bit 16 and the underreamer 18 are cutting the geological formation 14. In the example illustrated by Equation (9), because one data point (e.g., at depth 2) is measured between at depth 1 (e.g., when the drill bit 16 starts drilling) and depth 3 (e.g., when both the drill bit 16 and the underreamer 18 start drilling), there is one zero between $A_B$ and $A_U$ in the third row. Likewise, there is one zero between $A_B$ and $A_U$ in the following rows. It should be noted, however, Equation (9) may include any number of zeros between $A_B$ and $A_U$, when both are present, in each row, such as 0, 1, 2, 3, 4, 5, 6, 7, 8, 9, 10, and so forth. The number of zeros between $A_B$ and $A_U$ in each row depends at least on the number of data collection points in a depth range equal to the distance, $\delta$, between the drill bit 16 and the underreamer 18.

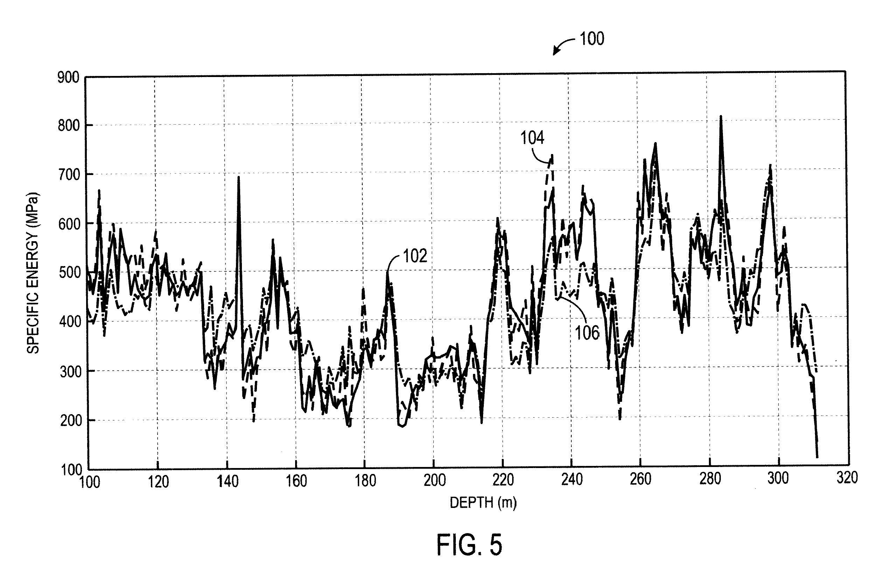

The techniques described herein for separating the specific energy at different depths into components coming from the drill bit 16 and the underreamer 18 operate without how the total weight, *W,* and the total torque, *T* is split between the drill bit 16 and the underreamer 18. In addition, the techniques described herein operate without obtaining the specific energy at each depth by assuming that the specific energies of the geological formation 14 at the drill bit 16 and at the underreamer 16 are the same. FIG. 5 is a chart 100 illustrating the specific energy as a function of depth of the wellbore 20 obtained using different techniques. More specifically, the chart 100 includes a first curve 102 (solid line) denoting true specific energies of the geological formation 14 at different depths of the wellbore 20. The first curve 102 may be used as a reference to compare with. A second curve 104 represents specific energies obtained in accordance with the techniques described herein. As illustrated, the second curve 104 generally overlaps with the first curve 102. A third curve 106 (dash-dot line) represents specific energies obtained using a common method. The common method essentially assumes that the specific energy at the drill bit 16 and at the underreamer 18 are the same and uses only Equation (1) to derive the specific energy. As illustrated, the third curve 106 does not overlap with the first curve 102 as well as the second curve 106 does.

The method according to the disclosure has been explained relative to the specific energy. However, other properties may be determined thanks to the aforementioned method as will be described in further details below. It may also be noted that the measured parameters (torque and weight on bit) may be measured not by the MWD tool 40 situated downhole but by a surface equipment.

A property that may also be determined is a property relative to a quantity of an element released from the formation. As noted above, the formation fluid stored in the geological formation 14 may be released by drilling the geological formation 14 from both the drill bit 16 and the underreamer 18. The formation fluid is then carried by the drilling fluid 32 to the surface 24. At the surface 24, the released formation fluid may be measured to determine various properties thereof and/or properties of the geological formation 14 that stored the formation fluid, such as the quantity of the formation fluid. But the formation fluid measured at the surface may combine formation fluid from the geological formation 14 drilled by both the drill bit 16 and the underreamer 18. As such, the quantity of the formation fluid measured at the surface, as represented by the first curve 82 in FIG. 3, may not correlate well with the true quantity of the formation fluid stored in the geological formation 14, as represented by the second curve 84 in FIG. 3.

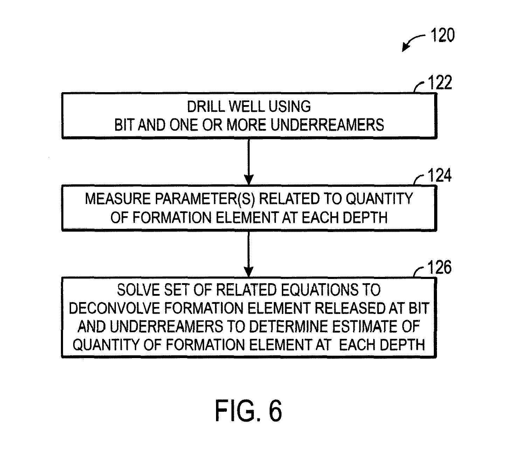

FIG. 6 illustrates a method 120 for estimating the quantity of an element released from the geological formation 14 into the drilling fluid 32, such as a quantity of a chemical component (e.g., an alkane) of the formation fluid. This element originates from the geological formation 14 at multiple depths of the wellbore 20 drilled with the BHA 28 that includes the drill bit 16 and the underreamer 18. The method may be used to determine the composition of the formation fluid by comparing the quantity of the respective measurement chemical component. The method may also apply to determine a quantity of the formation fluid released and/or cuttings drilled from the geological formation 14. The method 120 may start with drilling the wellbore 20 using the drill bit 16 and one or more underreamers (block 122). At each depth of the wellbore 20, one or more parameters related to the quantity of the formation element from the geological formation 14 may be measured (block 124). As the formation element may be carried by the drilling fluid 32 to the surface 12, the parameters may be measured at the surface 24 by the surface measurement system 52.

At each depth, the quantity of the formation element may be expressed in an equation, as discussed in more detail below, as a function of the measured parameters. Also as noted above, the quantity of the formation element from the geological formation 14 at each depth generally includes components coming from the drill bit 16 at that depth and the underreamer 18 at a smaller depth because of a distance between the drill bit 16 and the underreamer 18 (e.g., the distance 76 as illustrated in FIG. 3). As such, a set of related equations about the quantity of the formation element may be constructed. For example, if the drill bit 16 drills from a first depth to a second depth with the second depth is greater than the first depth by the distance between the drill bit 16 and the underreamer 18, the quantity of the formation element at the second depth is related to the quantity of the formation element at the first depth because the underreamer 18 is at the first depth when the drill bit 16 advances to the second depth. As such, the equations for the quantity of the formation element at the first and the second depths are related. The method 120 thus includes solving the set of related equations to deconvolve formation element quantity components coming from the drill bit 16 and the underreamer 18 to determine an estimate of the actual (true) quantity of the formation element from the geological formation 14 at each depth (block 126).

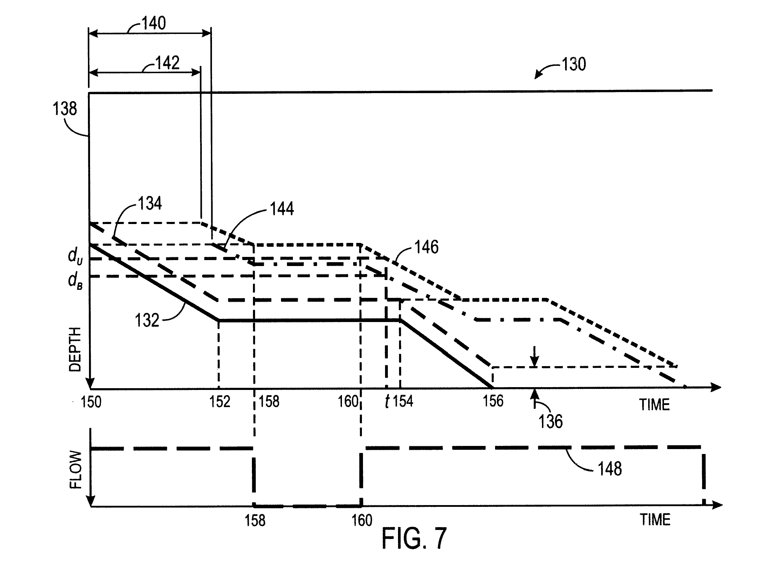

FIG. 7 is a chart 130 illustrating depths of the bit 16 and the underreamer 18, tracked based on at least a flow of the formation element at the drill bit 16 and the distance between the drill bit 16 and the underreamer 18, as a function of time. The chart 130 includes a first curve 132 (solid line) illustrating the actual depth of the drill bit 16 as a function of time and a second curve 134 (dash-dot line) illustrating the actual depth of the underreamer 18 as a function of time. The first curve 132 and the second curve 134 are parallel with one another with a constant distance 136 (along the depth axis 136) separating one another. The constant distance 136 is the distance between the drill bit 16 and the underreamer (e.g., the distance 76 as illustrated in FIGS. 2 and 5). As illustrated, both the drill bit 16 and the underreamer 18 drill between time 150 and time 152, rest between time 152 and 154, and continue drilling between time 154 and 156.

As noted above, the drilling fluid 32 may be pumped downward through the center of the drill string 26 to the drill bit 16 and return back to the surface 24 carrying therewith the drill cuttings and the formation fluid from both the drill bit 16 and the underreamer 18 through the annulus 33 between the drill string 26 and the geological formation 14. As it takes time for the drilling fluid 32 (with the drilling cuttings and the released formation fluid) to return back to the surface 24, where the drilling fluid 32 may be collected and analyzed, there is a time lag (e.g., time lag 140) between when the drill bit 16 was at a first predetermined depth and when the drilling fluid 32 that was at the drill bit 16 and extracted from the first predetermined depth returns to the surface 24. Likewise, there is a time lag (e.g., time lag 142) between when the underreamer 18 was at a second predetermined depth and when the drilling fluid 32 that was at the underreamer 18 and extracted from the first predetermined depth returns to the surface 24. The time lags 140, 142 depend at least on the flow rate of the drilling fluid 32, which in turn depends partly on the borehole size of the wellbore 20, and the actual depths of the drill bit 16 (e.g., the first curve 132) and the underreamer 18 (e.g., the second curve 134). The time lag 142 for the drill bit 16 is generally greater than the time lag 140 for the underreamer 18 as the drilling fluid 32 that was at the drill bit 16 travels a longer distance than the drilling fluid that was at the underreamer 18 before returning to the surface 24.

Therefore, by analyzing the return drilling fluid 32, which may carry the formation element released from the geological formation 14 at the drill bit 16 and the underreamer 18, at the surface 24, the depths of the geological formation 14 that generated the formation element at the drill bit 16 and the underreamer 18 may be determined or measured at a given time. A third curve 144 illustrates the depth of the geological formation 14 that generated the formation element at the drill bit 16 when the drilling fluid 32 that was at the drill bit 16 returns to the surface 24. Likewise, a fourth curve 146 illustrates the depth of the geological formation 14 that generated the formation element at the underreamer 18 when the drilling fluid 32 that was at the underreamer 18 returns to the surface 24.

The chart 130 also includes a fifth curve 148 illustrating the flow rate of the drilling fluid 32 as a function of time. As illustrated, the drilling fluid 32 flows in a constant rate except between time 158 and 160 during which the drilling fluid 32 stops. As noted above, measuring the depths of the drill bit 16 and the underreamer 18 by tracking the drilling fluid 32 depends partly on the flow rate of the drilling fluid 32. As such, each of the third curve 144 and the fourth curve 146 includes a flat region of measured depth between time 158 and 160, during which the drilling fluid 32 stops flowing. It should be noted that all the curves illustrated in FIG. 7 are nonlimiting examples for illustrating the measurement of depths of the geological formation 14 that generated formation element at the drill bit 16 and the underreamer 18, and that the techniques disclosed herein applies to any wellbore drilling, which may have different flow curves for the drilling fluid, different wellbore profiles (e.g., borehole size), different drilling profiles (e.g., drilling depth as a function of time), or different measured depths profiles (e.g., measured depth of geological formation 14 by tracking the drilling fluid 32 as a function of time), or any combination thereof.

As illustrated in FIG. 7, at a given time, t, the formation fluid measured at the surface 24 includes the formation fluid released from the geological formation 14 drilled by the drill bit 16, which was at depth $d_B$, and the formation fluid released from the geological formation 14 drilled by the underreamer 18, which was at depth $d_U$. A property, $h$, such as a quantity, of the formation element measured at the surface 24 at the given time, t, may be written as:

$$h(t) = A_B g(d_B) + A_U g(d_U) \tag{10},$$

where $A_B$ and $A_U$ are the cross-sectional areas of the geological formation 14 (e.g., rock) cut by the drill bit 16 and the underreamer 18, respectively, and g is a property of the formation element to be obtained or desired and is a function of depth, $d$, of the geological formation 14 (e.g., $d_B$ and $d_U$). The desired property, $g(d)$, of the formation element may include any property of the formation element, such as a quantity of the formation element in a unit area of the geological formation 14, or a quantity of the formation element (e.g., a certain chemical) contained in the formation element in a unit area of the geological formation 14. The property, $h(t)$, of the formation element measured at the surface 24, as noted above, is mixed with contributions from both the drill bit 16 and the underreamer 18 and may be any suitable property related to $g(d)$, such as a total quantity of the formation element measured at the surface 24 at any given time, or total quantity of a certain chemical contained in the formation element measured at the surface 24 at any given time.

Equation (10) may be written for different depths and time. For example, when the drill bit 16 starts to drill the geological formation 14 at a first depth (e.g., depth 1) and formation fluid starts to be released from the geological formation, the underreamer 18 is not yet drilling out any formation fluid. At a certain time later (e.g., time 1), the released formation fluid that was drilled from the geological formation 14 at depth 1 by the drill bit 16 returns to the surface 24 (e.g., carried by the drilling fluid 32) and is analyzed to obtain the property, $h$. Equation (10) may be written as:

$$h(1) = A_B g(1) \tag{11}.$$

At any depth (e.g., depth 2) where the underreamer 18 is not reaching depth 1, Equation (10) may be written similarly to Equation (11). As the underreamer 18 advances downward to reach depth 3, the underreamer 18 drills geological formation 14 to release more formation fluid. In the meantime, the drill bit 16 reaches to a depth (e.g., depth 3) that is deeper than depth 1 by substantially the distance, $\delta$, between the drill bit 16 and the underreamer 18. If the drill bit 16 also releases the formation fluid at depth 3, Equation (10) at a certain time later (e.g., time 3) becomes:

$$h(3) = A_B g(3) + A_U g(1) \tag{12}.$$

At any depth that is greater than depth 3 (e.g., depth 4, 5, 6, and so forth), both the drill bit 16 and the underreamer 18 are drilling the geological formation 14 and may release more formation fluid to be carried by the drilling fluid 32 back to the surface to be analyzed, and Equation (10) may be written similarly to Equation (12). Accordingly, a set of equations (e.g., including Equations (11) and (12)) based on Equation (10) may be constructed for different depths and time. The property, g, of the formation element at different depths may then be solved by solving the set of equations. As such, the measured property, $h$, of the formation element at the surface 24 at any given time may be deconvolved or decomposed to separate components coming from the geological formation 14 drilled by the drill bit 16 and by the underreamer 18.

In accordance with the present disclosure, solving for the set of equations based on Equation (10) (e.g., including Equations (11), (12), and so forth) may use any suitable means, such as least squares methods. For example, a matrix equation may be constructed based on Equation (10), or more specifically, Equations (11), (12), and so forth:

$$
\begin{bmatrix}
h(1) \\
h(2) \\
h(3) \\
\vdots \\
h(t-2) \\
h(t-1) \\
h(t)
\end{bmatrix}
=
\begin{bmatrix}
A_B & 0 \\
0 & A_B & 0 \\
A_U & 0 & A_B & 0 \\
& \ddots & \ddots & \ddots \\
& 0 & A_U & 0 & A_B & 0 \\
& & 0 & A_U & A_B & 0 \\
& & 0 & A_U & 0 & A_B & 0 \\
& & 0 & A_U & 0 & 0 & A_B \\
& & & 0 & A_U & 0 & A_B
\end{bmatrix}
\begin{bmatrix}
g(1) \\
g(2) \\
g(3) \\
\vdots \\
\\
g(d-2) \\
g(d-1) \\
g(d)
\end{bmatrix}
\qquad (13).
$$

In Equation (13), the property, $h$, of the formation element may be measured at the surface 24 at different time (e.g., time 1, 2, 3, ... $t$-2, $t$-1, $t$) and may be written as a first vector on the left side. The right side of Equation (13) includes a matrix and a second vector. The matrix includes non-zero elements of the cross-sectional area of the geological formation 14 (e.g., rock) cut by the drill bit 16, $A_B$, and the underreamer 18, $A_U$, respectively, which may be measured or known. As such, the second vector including the property, $g$, of the formation element at different depths (e.g., depth 1, 2, 3, ..., $d$-2, $d$-1, $d$) may be determined using linear algebra. Contrary to the previous embodiment, even though distance between the bit and the underreamer does not vary during drilling the measurement taken at a predetermined time may come from two depths separated by a distance that is varying, in function of the management of the well as explained above and visible in the matrix.

As illustrated by the third and fourth curves 144, 146 of FIG. 7, a given time, t, correlates with a depth of the drill bit 16, $d_B$, and a depth of the underreamer 18, $d_U$, by tracking the drilling fluid 32. Accordingly, the presence or absence of $A_B$ and $A_U$, as well as the positions of $A_B$ and $A_U$ in each row (e.g., the number of zeros before, after, and between $A_B$ and $A_U$) of the matrix in Equation (13) is determined by the depth of the drill bit 16, $d_B$, and depth of the underreamer 18, $d_U$, respectively, as a function of time.

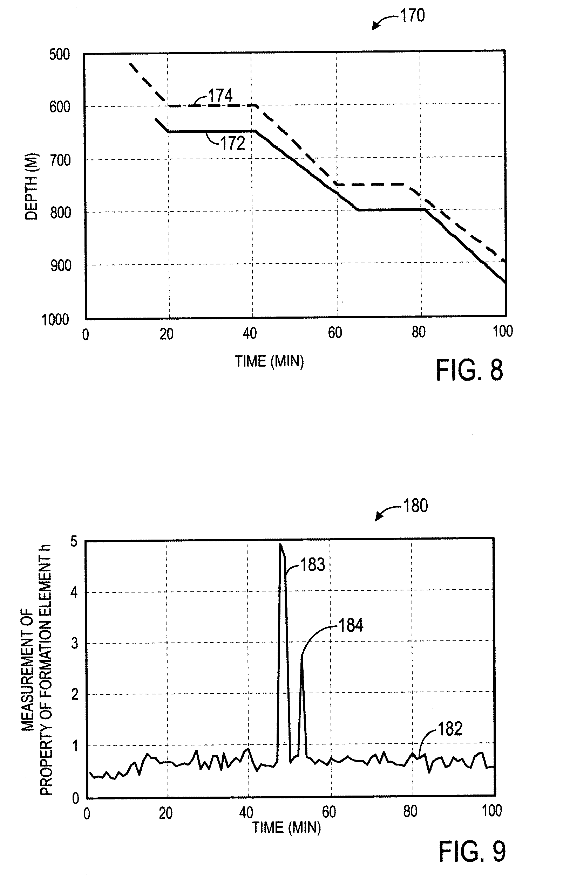

The techniques described herein for separating a property (e.g., property $h$) of the formation element measured at the surface 24 at any given time into components coming from the formation element released by the geological formation 14 drilled by the bit 16 and by the underreamer 18 may provide better estimation of a related property (e.g., property g) of the formation element at multiple depths. FIGS. 8-10 illustrate a process and results of the techniques described above. More specifically, FIG. 8 is a chart 170 illustrating a first curve 172 for depth of the drill bit 12 when the drilling fluid 32 (carrying the formation element released from the geological formation 14 drilled by the drill bit 12) returns to the surface 24, and a second curve 174 for depth of the underreamer 18 when the drilling fluid 32 (carrying the formation element released from the geological formation 14 drilled by the underreamer 18) returns to the surface 24. As noted above, the first curve 172 and the second curve 174 may be used to determine the positions of $A_B$ and $A_U$ in the matrix of Equation (13). It should be noted that the first curve 172 and the second curve 174 as illustrated in FIG. 8 are meant to be examples only, as the drilling system 10 may have different depth-time curves for the drill bit 16 and the underreamer 18, which, as discussed above, depend at least on the flow rate of the drilling fluid 32, the wellbore profile, and the actual depths of drill bit 16 and the underreamer 18.

FIG. 9 is a chart 180 illustrating measurement at the surface 24 of the property, $h$, of the formation element as a function of time (curve 182). As illustrated, the curve 182 includes two peaks 183, 184 of the measured property that are close to one another with respect to time. For example, the measured property, $h$, may be the total quantity of the formation element measured at the surface 24 from drilling the wellbore 20 with the BHA 28 that includes the drill bit 16 and the underreamer 18.

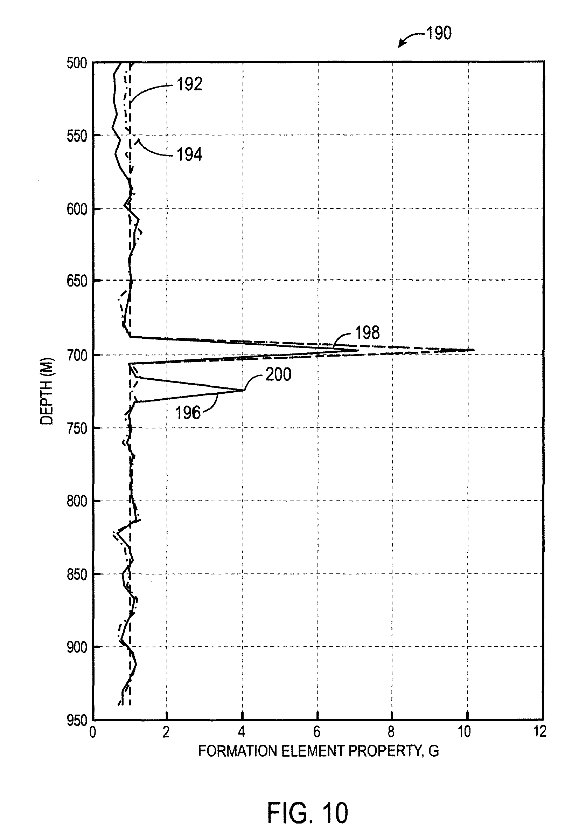

FIG. 10 is a chart 190 illustrating the property, g, of the formation element as a function of depth of the wellbore obtained using different techniques. More specifically, the chart 190 includes a first curve 192 (dashed line) denoting true property, g, of the formation element stored in the geological formation 14 at different depths of the wellbore 20. The first curve 192 may be used as a reference to compare with. A second curve 194 (dashed-dot line) represents the property, $g$, of the formation element derived from the depth-time curves 172, 174, as illustrated in FIG. 8, and the measured property-time curve 182, as illustrated in FIG. 9 with the techniques described herein. As illustrated, the second curve 194 generally overlaps with the first curve 192. A third curve 196 (solid line) represents the property, *g*, of the formation fluid obtained using a common method. Instead of separating the measured property, *h*, into components from the drill bit 16 and the underreamer 18, the common method essentially attribute the measured property, *h*, to only the drill bit 16. As the measured property, *h*, shows two peaks 183, 184 in the example illustrated in FIG. 9, the third curve 196 obtained from the common method shows two peaks 198, 200 as well, which does not correlate well with the first curve 192.

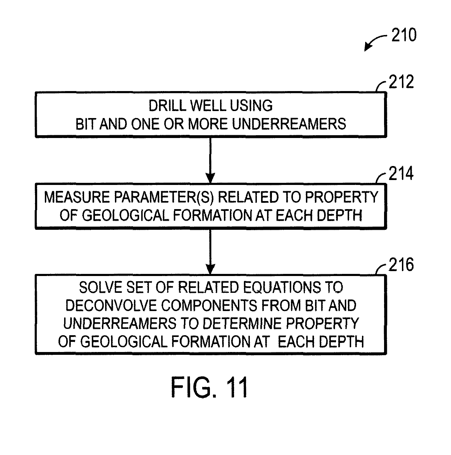

As noted above, the techniques described herein applies generally to any properties of the geological formation 14 drilled with the BHA 28 that includes the drill bit 16 and one or more underreamers 18. The properties of the geological formation 14 may include the specific energy of the geological formation 14 at multiple depths of the wellbore 20 and the properties (e.g., the quantity of the formation element) of formation fluid stored in the geological formation 14 at multiple depths of the wellbore 20. FIG. 11 illustrates a method 210 for separating components of measured parameter(s) coming from both the drill bit 16 and the underreamer 18 to determine properties of the geological formation 14 at multiple depths of the wellbore 20, in accordance with the present disclosure. The method 210 may start with drilling the wellbore 20 using the drill bit 16 and one or more underreamers (block 212). At each depth of the wellbore 20, one or more parameters (e.g., volume, weight, pressure, specific energy, quantity of stored formation fluid, quantity of certain chemicals in the stored formation fluid, and so forth) related to a property of the geological formation 14 may be measured (block 214), for example, from well-logging data. The one or more parameters may be measured below the surface 24 (e.g., by one or more downhole tools, such as the MWD tool 40 of the BHA 28), and/or at the surface 24 (e.g., by the surface measurement system 52).

At each depth, the property of the geological formation 14 may be expressed in an equation as a function of the measured parameters. The equation may include separate terms illustrating components from the drill bit 16 and the underreamer 18. As such, a set of related equations about the property of the geological formation may be constructed. Solving the set of related equations may be performed with any suitable methods to deconvolve the components of the property of the geological formation 14 drilled by the drill bit 16 and by the underreamer 18 to determine an estimate of the actual (true) property of the geological formation 14 at each depth (block 216).

The specific embodiments described above have been shown by way of example, and it should be understood that these embodiments may be susceptible to various modifications and alternative forms. It should be further understood that the claims are not intended to be limited to the particular forms disclosed, but rather to cover modifications, equivalents, and alternatives falling within the spirit and scope of this disclosure.

**Claims**

1.  A method comprising:

    receiving a set of measurements corresponding to mixed well-logging data relative to a geological formation at multiple depths of a wellbore drilled using a bottom hole assembly (BHA) that includes a drill bit and a first underreamer, wherein the drill bit and the first underreamer are spaced a first distance apart from one another;
    separating the set of measurements into first well-logging data corresponding to the geological formation at depths drilled by the drill bit and second well-logging data corresponding to the geological formation at depths reamed by the first underreamer; and
    determining a property relative to the geological formation on the basis of the separated measurements.

2.  The method of claim 1, wherein the set of measurements comprises measurements made by a downhole tool below surface or by a measurement device at surface.

3.  The method of claim 2, wherein the set of measurements comprises specific energy of the geological formation at the multiple depths.

4.  The method of claim 2, wherein the set of measurements comprises analysis of an element released from the formation into a drilling fluid.

5.  The method of claim 4, wherein the set of measurements comprises volume of cuttings extracted from the formation, quantity of a component of a formation fluid released from the wellbore, composition of the formation fluid, or any combination thereof.

6.  The method of claim 1, wherein separating the set of measurements comprises solving a set of equations for a property of the geological formation at the multiple depths of the wellbore.

7. The method of claim 6, wherein at least one equation of the set expresses a measurement in function of the property at least at two different depths.

8. The method of claim 7 in combination with any of claims 4 and 5, comprising calculating a lag time for the drilling fluid wherein the different depths expressing the measurement are determined based on the lag time calculation.

9. The method of any of claims 6 to 8, wherein solving the set of equations comprises constructing the set of equations into a matrix equation and solving the matrix equation for the property of the geological formation at the multiple depths, wherein the matrix equation comprises a matrix of coefficients, wherein the coefficients comprise cross-sectional areas of the geological formation cut by the drill bit and the underreamer.

10. The method of any of the preceding claims, comprising plotting the property in function of depth based on the separation of measurements into first and second well-logging data.

11. A drilling assembly comprising:

a bottom hole assembly (BHA) comprising a drill bit and a first underreamer, wherein the drill bit and the first underreamer are spaced a first distance apart from one another; and
a controller comprising a processor programmed to:

receive a set of measurements corresponding to mixed well-logging data relative to a geological formation at multiple depths of a wellbore drilled using the BHA;
separate the set of measurements into first well-logging data corresponding to the geological formation at depths drilled by the drill bit and second well-logging data corresponding to the geological formation at depths reamed by the first underreamer; and
determine a property relative to the geological formation on the basis of the separated measurements.

12. The drilling assembly of claim 11, wherein the BHA comprises a second underreamer, wherein the second underreamer is spaced apart from the first underreamer a second distance and spaced apart from the drill bit a third distance, and wherein the processor is programmed to separate the set of measurements into the first well-logging data corresponding to the geological formation at depths drilled by the drill bit, the second well-logging data corresponding to the geological formation at depths reamed by the first underreamer, and third well-logging data corresponding to the geological formation at depths reamed by the second underreamer.

13. The drilling assembly of claim 11 or 12, comprising a downhole tool below surface, a measurement device at surface, or a combination thereof, configured to obtain the set of measurements.

14. The drilling assembly of any of the claims 11 to 13, wherein the processor is programmed to separate the set of measurements by solving a set of equations, each equation includes a property of the geological formation at a depth of the multiple depths represented by a first component attributable to the drill bit and a second component attributable to the underreamer.

15. The drilling assembly of any of the claims 11 to 14, wherein the processor is programmed to receive the set of measurements corresponding to the mixed well-logging data relative to the geological formation at the multiple depths with an increment of depth that is less than the first distance.

FIG. 1

FIG. 2

FIG. 3

90

92

DRILL WELL USING BIT AND
ONE OR MORE UNDERREAMERS

94

MEASURE PARAMETER(S) RELATED
TO SPECIFIC ENERGY AT EACH DEPTH

96

SOLVE SET OF RELATED EQUATIONS TO
DECONVOLVE SPECIFIC ENERGY
OF BIT AND UNDERREAMERS TO
DETERMINE ESTIMATE OF TRUE
SPECIFIC ENERGY OF EACH DEPTH

FIG. 4

FIG. 5

120

122

DRILL WELL USING
BIT AND ONE OR MORE UNDERREAMERS

124

MEASURE PARAMETER(S) RELATED TO QUANTITY
OF FORMATION ELEMENT AT EACH DEPTH

126

SOLVE SET OF RELATED EQUATIONS TO
DECONVOLVE FORMATION ELEMENT RELEASED AT BIT
AND UNDERREAMERS TO DETERMINE ESTIMATE OF
QUANTITY OF FORMATION ELEMENT AT EACH DEPTH

FIG. 6

FIG. 7

EP 3 012 671 A1

FIG. 8

FIG. 9

FIG. 10

~210

~212

DRILL WELL USING
BIT AND ONE OR MORE UNDERREAMERS

~214

MEASURE PARAMETER(S) RELATED TO PROPERTY
OF GEOLOGICAL FORMATION AT EACH DEPTH

~216

SOLVE SET OF RELATED EQUATIONS TO
DECONVOLVE COMPONENTS FROM BIT AND
UNDERREAMERS TO DETERMINE PROPERTY
OF GEOLOGICAL FORMATION AT EACH DEPTH

FIG. 11

Europäisches
Patentamt

European
Patent Office

Office européen
des brevets

**EUROPEAN SEARCH REPORT**

Application Number

EP 14 29 0323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/211470 A1 (JEFFRYES BENJAMIN P [GB] JEFFRYES BENJAMIN PETER [GB]) 29 September 2005 (2005-09-29) | 1-3,6, 10-15 | INV. G01V9/00 E21B49/00 G01N33/28 |
| A | * abstract; figure 1 * * paragraphs [0002], [0003], [0011] - [0023], [0050] - [0054], [0062], [0063], [0087] - [0092] * ----- | 4,5,8 | ADD. G01V1/40 |
| X | US 2011/232968 A1 (PURWANTO ARIFIN [US] ET AL) 29 September 2011 (2011-09-29)  * abstract; figures 1-4,7 * * paragraphs [0002], [0003], [0009], [0010], [0028] - [0042] * ----- | 1-3,6,7, 9,10,12, 14,15 | |
| X | US 2010/133008 A1 (GAWSKI VICTOR [GB] ET AL) 3 June 2010 (2010-06-03)  * abstract; figures 3,9,12-14 * * paragraphs [0159], [0178] - [0181] * ----- | 1-3,6, 10,11, 14,15 | |
| X | WO 2014/105034 A1 (HALLIBURTON ENERGY SERV INC [US]) 3 July 2014 (2014-07-03) * abstract; figure 1 * * paragraphs [0004], [0009], [0010] * ----- | 1,11 | TECHNICAL FIELDS SEARCHED (IPC)  G01V E21B G01N |
| A | US 4 739 655 A (GREER CARL T [US] ET AL) 26 April 1988 (1988-04-26) * abstract; figures 1-3 * * column 1, lines 15-26 * * column 2, lines 52-59 * * claims 1,3 * ----- | 4,5,8 | |
| A | US 4 860 836 A (GUNTHER LARRY J [US]) 29 August 1989 (1989-08-29) * abstract; figures 1-4 * * column 1, line 43 - column 3, line 35 * * column 11, line 57 - column 12, line 28 * ----- | 4,5,8 | |

The present search report has been drawn up for all claims

| Place of search | Date of completion of the search | Examiner |
|---|---|---|
| The Hague | 24 April 2015 | de Jong, Frank |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...............................................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 14 29 0323

This annex lists the patent family members relating to the patent documents cited in the above-mentioned European search report.
The members are as contained in the European Patent Office EDP file on
The European Patent Office is in no way liable for these particulars which are merely given for the purpose of information.

24-04-2015

| Patent document cited in search report | Publication date | Patent family member(s) | Publication date |
|---|---|---|---|
| US 2005211470 A1 | 29-09-2005 | BR PI0500981 A<br>CA 2502165 A1<br>GB 2412388 A<br>GB 2412392 A<br>NO 327621 B1<br>US 2005211470 A1 | 08-11-2005<br>27-09-2005<br>28-09-2005<br>28-09-2005<br>07-09-2009<br>29-09-2005 |
| US 2011232968 A1 | 29-09-2011 | NONE | |
| US 2010133008 A1 | 03-06-2010 | CA 2659453 A1<br>GB 2457604 A<br>MY 151779 A<br>US 2010133008 A1<br>WO 2008039523 A1 | 03-04-2008<br>26-08-2009<br>14-07-2014<br>03-06-2010<br>03-04-2008 |
| WO 2014105034 A1 | 03-07-2014 | NONE | |
| US 4739655 A | 26-04-1988 | NONE | |
| US 4860836 A | 29-08-1989 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7232548 B